# EUROPEAN PATENT SPECIFICATION

(11) **EP 2 768 575 B1**
(45) Date of publication and mention of the grant of the patent: **26.02.2020**
(21) Application number: 12840943.0
(22) Date of filing: 19.10.2012
(51) Int. Cl.: A61N 1/00, A61N 1/36, A61N 1/04

(54) **AUTOMATED ELECTRODE ARRAY TREATMENT PROTOCOL**
AUTOMATISIERTES BEHANDLUNGSPROTOKOLL FÜR ELEKTRODENANORDNUNG
PROTOCOLE DE TRAITEMENT AUTOMATISÉ DE RÉSEAU D'ÉLECTRODES

(30) Priority: 19.10.2011 US 201161627867 P
(43) Date of publication of application: 27.08.2014
(73) Proprietor: HTK Enterprises, Inc., Richardson, TX 75081 (US)
(72) Inventor: MAGEE, Paul J., Kings Langley Hertfordshire WD4 9HS (GB)
(74) Representative: Uexküll & Stolberg
(86) International application number: PCT/US2012/061163
(87) International publication number: WO 2013/059699

(56) References cited:
- WO-A1-2009/135693
- WO-A1-2010/064206
- US-A1- 2007 129 759
- US-A1- 2007 250 119
- US-A1- 2009 118 790
- US-A1- 2009 281 601
- US-A1- 2010 087 903
- US-A1- 2011 137 381

## Description

### TECHNICAL FIELD

The disclosure relates generally to an automated protocol and a device for treating a patient with the electrode array.

### BACKGROUND

An objective of transcutaneous neurostimulation is to focus an adequate electrical energy concentration at a relatively small, preferred treatment location on the skin. The preferred treatment locations are typically locations such as nerve branches, trigger points, and acupuncture points, and are evidenced as points of lower relative impedance.

One approach to determine the location of low relative impedance points is to use an array of electrodes where the pairs of alternating polarity are individually addressable. This method is employed by Bijelic in U.S. Patent Application Serial No. 2008/0027507. An operator can then step through the combinations of electrodes, scanning for low relative impedance values. After the low impedance points are identified, the operator must use another device to treat the identified areas. The process of scanning and treating must be repeated to accomplish the desired results. Performing this approach, either manually or in an automated fashion, is time-consuming and costly.

Other prior devices and methods do not address the problem. For example, in U.S. Pat. No. 4,238,726, Ichijo discusses a method of determining points of low impedance electrically without mentioning using an electrode configuration as a means. Molina-Negro et al., in U.S. Pat. No. 4,541,432, discuss using a waveform to treat pain without regard to the electrode configuration. Matos, in U.S. Patent Application Serial No. 2003/0233129, discusses a method of scanning and stimulating without regard to relative electrode size and spacing.

Another common method of locating low impedance points is to use a configuration of concentric electrodes. An outer electrode has a substantially larger area than a smaller, inner "treatment" electrode. The area ratio of the outer electrode to the inner electrode is typically between 1,2/1 and 5,0/1, and the spacing between the electrodes is controlled at a small dimension, which is typically less than 0.40 inches.

By measuring and noting the impedance measurements between the inner and outer electrodes, as the electrode pair is moved over the body, one can locate points of low impedance and, thus, preferred treatment locations. This is discussed generally by Colthurst in U.S. Pat. No. 7,483,734.

Axelgaard, in U.S. Pat. No. 6,038,485, discusses controlling current distribution and directing electrical pulses via rows and columns of electrodes. Axelgaard addresses spacing and area of individual electrodes but does not disclose anything but equal spacing and equal electrode areas. Schumann, in U.S. Patent Application Serial No. 2007/0106342, discusses a means of scanning and locating trigger points and mentions a relationship between spacing and skin conductance. However, Schumann does not teach anything about having different areas of electrodes in combination with particular spacing between the electrodes. Schumann also discusses "chasing the pain" via multiple cycles of scanning and treating. Also worth noting is that Bijelic, in U.S. Patent Application Serial No. 2008/0027507, discusses arrays of electrodes with a single spacing and single dimension. U.S. Patent Application Serial No. 2010/087903 provides an electrotherapeutic device for application on a human or animal body part, comprising electrodes, a measuring means connected thereto and arranged to measure a value of an electrical quantity, and a control unit to process the measured values, wherein the control unit further comprises pattern recognition means to determine a pattern in the measured values, for example by comparing with patterns stored in a memory unit. By means of the determined pattern, the device is able to know its position on the body, and to determine whether this position is suitable for a treatment, and to which electrodes a treatment signal must be supplied. The device is much easier to use since it does not require any user's knowledge about patterns, as this may be stored in the device itself. International Application WO 2010/064206 A1 relates to an electrical stimulation device and to a method of locating an electrical stimulation point of a human or animal. The electrical stimulation device comprises: a matrix of electrodes distributed on an electrode pad being configured to be applied on the human or animal body, the distribution of the electrodes in the matrix being arranged for covering a plurality of the stimulation points of the human or animal, an electronic circuit being connected to the electrodes of the matrix, and being configured for applying an electrical stimulation signal to the electrodes of the matrix, and feedback means for providing a feedback signal to the electrical stimulation device in response to the applied electrical stimulation signal. The electrical stimulation device being configured for sequentially applying the electrical stimulation signal to subsets of electrodes of the matrix, and for receiving the feedback signal in response to the applying of the electrical stimulation signal to the subsets of electrodes. The effect of the electrical stimulation device is that the electrical stimulation signal generates some kind of feedback signal which is used in assessing the suitability of the subset of electrodes as an electrical stimulation point of the human or animal. By sequentially applying the electrical stimulation signal to the subsets of electrodes, the suitability of the electrodes of the matrix of electrodes as electrical stimulation point is tested by the electrical stimulation device.

Once a treatment point is located, electrical energy may be applied to that point for treatment purposes. However, this is normally a manual process. If the discusses arrays of electrodes with a single spacing and single dimension.

Once a treatment point is located, electrical energy may be applied to that point for treatment purposes. However, this is normally a manual process. If the optimal treatment point changes, one must manually identify a new treatment point and apply treatment energy to the new point.

US 2011/0137381 A1 relates to treatment of neurological disorders via electrical stimulation, and methods related thereto. Disclosed are medical devices for the prevention and/or treatment of neurological disorders via electrical stimulation, and methods related thereto. The devices may also be utilized to detect disorders before the prevention or treatment of a neurological disorder. These devices are minimally or non-invasive. The present medical devices comprise various components, which include electrodes and control electronics. The electrodes are targeting electrodes constructed from ring type structures or virtually connected disc type arrays. The electrodes are located entirely outside the skull. The present medical devices also comprise one or more subsystems, a control system, a battery unit, and wires connecting the one or more subsystems. These devices may also be used for acute seizure control.

### SUMMARY

A treatment protocol is provided for use in conjunction with neurostimulation devices. Preferably, the treatment protocol provides unattended treatment. However, it should be understood that steps in the treatment process may be conducted manually.

It has been discovered that applying treatment energy to a treatment point changes the relative impedance reading of that point compared to surrounding points. This results in a user manually "chasing" a treatment point on a patient. It has also been discovered that pain or other ailments in one general location on a patient are not necessarily best treated by applying treatment energy to points within that general area. Rather, a better result is sometimes achieved by treating points in a different of changing an impedance value of one site to affect a relative impedance value of an adjacent site.

In accordance with at least certain example embodiments, treatment sites on the skin are identified automatically. An appropriate amount of treatment energy is focused at the site(s) to treat the sites effectively without over-treating.

In certain embodiments, the treatment protocol incorporates the use of an array of physical electrodes with alternating polarity electrodes, and whose spacing is held at a uniform distance. The distance is preferably less than 0.40 inches and the relative area of a treating electrode and the surrounding opposite polarity electrodes is preferably at least a ratio of 1,2/1. In some embodiments, the range of relative area is from about 1,2/1 to about 5,0/1. Stated another way, the relative area of a higher-voltage electrode (or electrode portion that is part of a virtual electrode pair) compared to a lower-voltage electrode (or electrode portion that is part of a virtual electrode pair) is preferably in the range of from about 1,2/1 to about 5,0/1. However, it should be understood that the polarity of the electrodes, virtual electrode pairs, and/or electrode nodes, as well as the relative area, may be reversed depending on the treatment objectives.

Such an array of electrodes then "self-selects" the optimal treatment electrodes at the preferred treatment location via Ohm's law since the greatest energy is delivered by the electrode located on the lowest impedance points. Moreover, the array "self-directs" treatment energy to the appropriate site within the array. Therefore, both scanning and treatment is performed automatically and concurrently. It should be understood that this is only an example of the device that may be employed in the treatment protocol and other neurostimulation devices may be used.

The electrode array device is used in the context of the treatment protocol, which preferably automates what would otherwise be a manual process. In one example embodiment, a method of neurostimulation is provided. The method includes establishing a first treatment area array having a plurality of sites. The method also includes scanning a plurality of sites of a treatment area array to determine an activity reading value for each of the plurality of sites. The method also includes determining a first subset of the plurality of sites of the treatment area array based on the activity reading value. The method also includes stimulating each site within the first subset of sites.

According to the present invention, a system is provided. The system includes an assessment apparatus operable to measure a first activity reading value of a plurality of sites within a treatment area array. The system also includes a computer operable to receive the first activity readings from the device, and to determine a first subset of the plurality of sites for stimulation.

In another example embodiment, a non-transitory computer-readable medium is provided that includes software executable by a processor. The software is operable to receive first activity reading values for a plurality of sites of a treatment area array. The software is also operable to determine a first subset of sites for stimulation from the plurality of sites of the treatment area array based on the first activity reading values. The software is also operable to receive second activity reading values for at least one site of the first subset after at least one site of the first subset has received electrical stimulation. The software is also operable to determine a second subset of sites for stimulation from the first subset of sites.

One or more of the embodiments may provide some, none, or all of certain of the following advantages. One advantage is that at least some manual assessment and treatment steps are automated. Another advantage is that at least some assessment and treatment steps may be achieved without at least some of the feedback from an electrode to a user or additional system component, which would be needed in typical systems.

### BRIEF DESCRIPTION OF THE DRAWINGS

For a more complete understanding of this disclosure and its features, reference is now made to the following description, taken in conjunction with the accompanying drawings, in which:
FIG. 1 is a flow chart illustrating an example protocol according to an example embodiment;
FIG. 2 is a screen shot illustrating an assessment of relative impedance values for a treatment area array associated with a physiological region according to an example embodiment;
FIG. 3 is a screen shot illustrating a treatment area array associated with a physiological region according to an example embodiment;
FIG. 4 is a screen shot illustrating assessment of a first segment associated with a treatment area array according to an example embodiment;
FIG. 5 is a screen shot illustrating assessment of a second segment associated with a treatment area array according to an example embodiment;
FIG. 6 is a screen shot illustrating an assessment of sites within a treatment area array at a step in a treatment protocol according to an example embodiment;
FIG. 7 is a screen shot illustrating the identification of certain sites within a treatment area array according to an example embodiment;
FIG. 8 is a screen shot illustrating re-assessment of certain sites within a treatment area array according to an example embodiment;
FIG. 9 is a screen shot illustrating identification and treatment of a primary active site within a treatment area array according to an example embodiment;
FIG. 10 is a screen shot illustrating a chart that may be used as part of at least one protocol according to an example embodiment;
FIG. 11 is a screen shot of a treatment area array associated with a facial region according to an example embodiment;
FIG. 12 is a screen shot illustrating assessment of a segment of a treatment area array associated with a facial region according to an example embodiment;
FIG. 13 is a screen shot illustrating assessment of all sites within a treatment area array associated with a facial region according to an example embodiment;
FIG. 14 is a screen shot illustrating treatment of active sites within segments of a treatment area array according to an example embodiment;
FIG. 15 is a screen shot illustrating interpreting results of at least one protocol according to an example embodiment;
FIG. 16 is a block diagram illustrating a system for executing at least one protocol according to an example embodiment;
FIG. 17 is a block diagram illustrating a computing platform for executing at least one protocol according to an example embodiment; and
FIG. 18 is a block diagram illustrating a network for executing at least one protocol according to an example embodiment.

### DETAILED DESCRIPTION

Certain embodiments provide treatment protocols for automating the neurostimulation treatment process. In certain examples, the protocol is an iterative process in which treatment points are first identified based on relative impedance readings, and then treated with application of treatment energy. Then the points are re-scanned and a subset of the points is re-treated. Certain other example protocols involve identifying physiological treatment regions that are different from a physiological region experiencing pain.

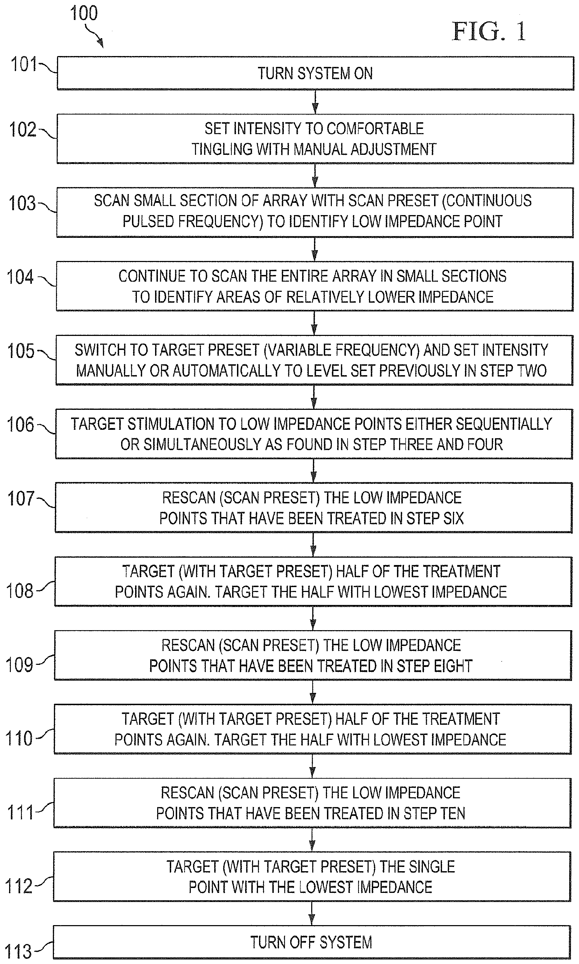

FIG. 1 is a flow chart illustrating an example treatment protocol. It should be understood that this is an example only and certain steps may be modified, added or eliminated, and that the order of certain steps may be changed. These modifications are within the scope of this disclosure and are within the knowledge of one having ordinary skill in the art. It should also be understood that the terminology used in the flow chart, and elsewhere herein, is not intended to be limiting.

As shown in FIG. 1, method 100 for a neurostimulation protocol is provided. In at least one embodiment the protocol is executed in the context of treating an ailment, such as pain, associated with a physiological region on a patient. In certain examples illustrated herein, this region is the back or the face of a human. The invention is not intended to be so limited, however. The protocols described herein may be applied to other physiological regions and to other patients (e.g., animals) and in other contexts. In the case of a human patient, for example, an example protocol may be applied to a first physiological region (e.g., the back). As a precursor to the method, or part of the method in certain embodiments, the physiological region is divided into an array of target areas or points. It should be understood that the terminology of "target areas or points" is not limiting. The divided sub-areas of the array may have any shape so long as each part of the array is a portion of the overall area covered by the array. Also, it is not required that the array encompass the entire physiological region being assessed and treated.

Method 100 has a first step 101, which comprises turning on a system operable to execute the protocol. Such a system is described elsewhere herein. According to step 102 an intensity level of an energy source is preset to a predetermined comfort level associated with assessment and treatment of a physiological region of a patient. According to step 103, a subsection of a treatment area array is scanned. The treatment area array is a division into subcomponents of a larger area associated with a physiological region being assessed and/or treated. The scanning is executed to determine a plurality of low impedance points within the scanned subsection. It should be understood that the term "point" refers to a specific point to treat or measure, as determined by relative readings, for example. "Site" may be used interchangeably with "point" herein. However, "site" in certain contexts refers to a sub-area within a treatment area array. Preferably, scanning is accomplished with a scan preset for which continuous pulsed frequency energy is delivered to the area, subcomponent, point, etc. being assessed or treated.

In some embodiments, the scanning is accomplished by a device operable to deliver the desired energy. This may be a hand-held device, for example, connected to an energy source. The device may also be connected to a computing device, which is operable to receive information from the device, store the information, manipulate the information, and automatically perform at least certain steps within the protocol. The device may also be an electrode array device, for example, which may be applied to the physiological region to at least partially cover the region. In such a context, it is preferable that each electrode node, or virtual node, correspond to one of the subcomponents of the treatment area array. In other words, the array of electrodes or electrode nodes may define the treatment area array.

In at least some embodiments the treatment area array subcomponents are scanned with a delivered energy source having characteristics amenable to identifying low impedance value areas without treating those areas. It should be recognized, however, that delivery of almost any energy amount (or type) may cause the relative impedance value of a given point to fluctuate. In at least some embodiments, scanning for low impedance points means scanning for subcomponents that have low impedance as compared to one or more adjacent or surrounding points or subcomponents.

As will be more apparent from the remaining description, in at least certain embodiments, an "activity reading" refers to an objective measurement of the effect that skin impedance has on a waveform of energy applied to that point. The activity reading may be used to provide a relative measurement of tissue impedance between distinct points on the skin. A higher activity reading may be used to indicate a low-impedance value or site, and thus where treatment is to be applied. "Point stimulation" or "point stim" refers to treatment of a specific area of pain and/or low impedance. When a point-stimulation is completed, a "point stimulation value" may be assessed. In certain cases, a site with a relatively higher activity reading indicates a relatively-lower skin impedance. Also, in certain cases, a point-stim value is indicated of a secondary activity reading. The description herein also reflects the concept of "multi-stim," which refers to a series of point-stims delivered to one treatment point.

According to step 104, additional subsections of the array are scanned to identify points or areas having low impedance. In at least some embodiments, a predetermined number of low impedance points are established. If that number is reached prior to scanning all of the subsections, then, as additional subsections are scanned, points having lower impedance replace points with higher impedance from the already-scanned subsections. Preferably all of the subsections or subcomponents of the entire treatment area array are scanned during this part of the process.

According to step 105, a target preset is selected. The target preset is associated with a delivered energy signal having a variable frequency. Also, in step 105 it is preferable to set an intensity value that is commensurate with the comfort level established earlier in step 102.

According to step 106, target stimulation (e.g., treatment energy selected and/or established in step 105) is delivered to the low impedance points identified earlier. In some embodiments, the treatment energy is delivered sequentially according to geographical positioning within the area, or according to the previously-determined relative impedance value, or according to some other criterion. In other embodiments, the treatment energy may be delivered to one or more first points, and then to one or more second points. The sets of points may be in order as described above, or may be in a randomly-selected order. In still other embodiments, two or more (or all) of the identified low impedance points may be treated simultaneously.

According to step 107, the low impedance points having been treated in step 106 are re-scanned (as in steps 103, 104) to determine new relative impedance values. This is done because the treatment step 107 will affect the relative impedance of the spot being treated.

In step 108, a subset of the low impedance points are treated again (as in step 106). The subset can be determined according to any number of suitable criteria. According to one aspect, a predetermined percentage is set and the number of low impedance points treated in this step is the predetermined percentage of the prior treated points. The exact points may be randomly selected until the percentage is reached. Alternatively, additional selection criteria may be incorporated. For example, if the percentage is 50%, then the 50% having the lowest impedance values (as measured in re-scanning step 107) may be treated. Other criteria may be used including preference based on geographic positioning. For example, points adjacent to, or within a certain distance of a particular spot may receive a weighting preference. The particular spot might be, for example, the spot of perceived pain, or the spot having the highest initial activity reading. In another alternate embodiment, a certain number of most-active (higher activity reading) points are selected. In following steps, the percentage, or number, of target points may be reduced all the way to a single point.

In step 109, the thus-treated points are now re-scanned. In step 110, a subset of the points rescanned in step 109 are treated. The re-scanning and re-treating steps are repeated in steps 111 and 112 until, preferably, a single point of lowest impedance is determined. This point may be again treated (not expressly shown). It should be noted that the re-treating steps illustrated in FIG. 1 involve re-treating half of the previously scanned (or re-scanned) points and that half represents the points having the lowest re-determined impedance values (e.g., higher re-determined activity readings). This is an example only and, as previously discussed, other criteria may be used to determine the number of, and particular ones of, the low impedance points that will receive treatment or re-treatment.

In step 113, the procedure is ended and the system is turned off. It should be noted that other protocols, methods, or steps described herein may, at this point be done or re-done. For example, a protocol which involves assessing and treating a facial treatment array area for perceived back pain may be executed after the assessment and treatment (or re-treatment) of the treatment area array associated with the back.

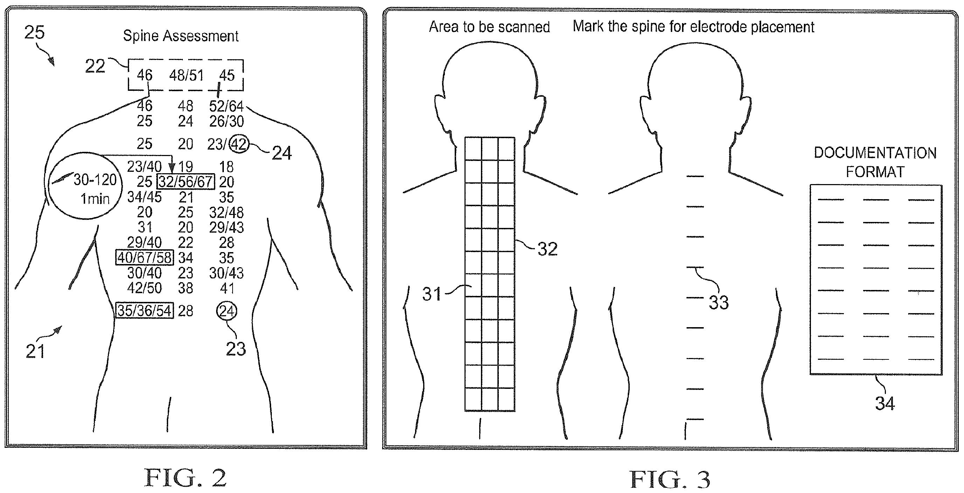

FIGS. 2 through 16 illustrate a treatment array area associated with back pain. Again, it should be noted that this is provided for example purposes only and is no way intended to limit the scope of the various embodiments disclosed and described herein. These features illustrate certain aspects associated with the protocol described in connection with FIG. 1, as well as some additional and/or modified aspects. It should also be noted that FIGS. 2-16 may also represent wire frames or screen shots as might be associated with a software program and/or a graphical user interface (GUI) as may provide the functionality described herein in accordance with one or more additional example embodiments.

FIG. 2 illustrates a physiological region 21, which in this example is a patient's back. Also illustrated is a treatment area array 25. It can be seen that the array is a 3 by 14 array, although arrays having different dimensions may be utilized. In this example there are 14 rows and 3 columns. Value 23 represents an example activity reading of a spot associated with the particular area on the back. Thus, item 23 can also be viewed as representing a particular subcomponent of the array. Dashed box 22 represents a segment or subsection of the array. It should be understood that segments or subsections may have different configurations and may include different numbers of subcomponents. The various values illustrated in FIG. 2 represent activity readings. It can be seen that at some points there are two or three values. For example, value 42 is a secondary reading after that point was initially assessed and treated. Thus all of the points that have two values were subjected to secondary readings according to, for example, the iterative process described in connection with FIG. 1. Those particular points were treated and then re-assessed. Points that have a third value were treated a second time and assessed a third time, and so on. It should be noted that the array developed by conducting primary assessments, secondary assessments, and so forth may be used as part of the automated protocol(s) described herein, or as an initial assessment tool to determine treatment areas, on which additional protocol arrays may be established.

FIG. 3 is another illustration of the physiological region being a patient's back. A treatment area array is shown as being divided into an actual grid 31. Activity readings would be measured for one or more of the blocks 32 defined by grid 31. Also illustrated are markings 33 on the physiological region. These markings represent target placement of electrodes for treatment and/or activity reading purposes. Also illustrated is a documentation grid 34 that may be used to record activity readings and/or point-stimulation values. Preferably, documentation grid 34 has data input fields that correspond, both in number and orientation, to the blocks 32 of grid 31 of the treatment area array.

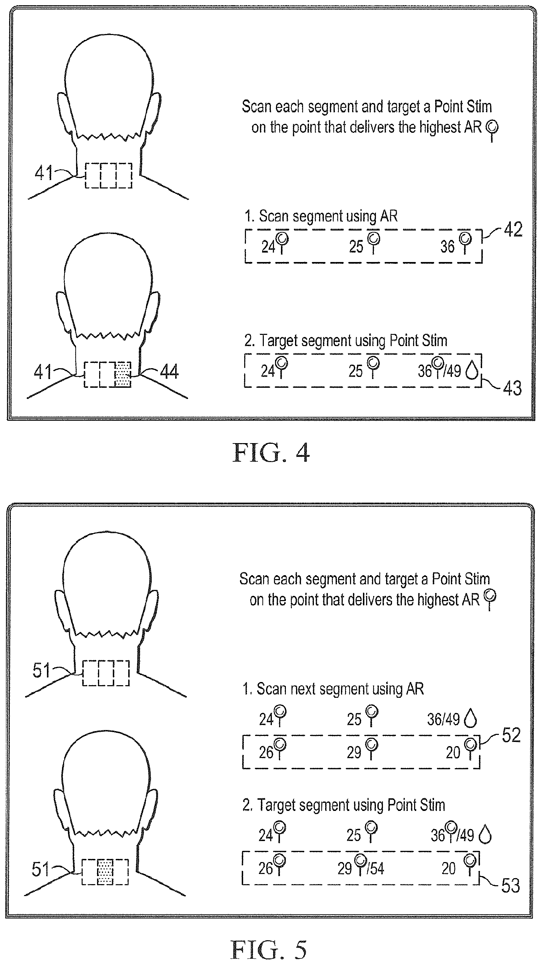

FIG. 4 illustrates the same physiological area. Item 41 is an illustration of a first segment (or subsection) of the treatment area array. Thus, this is one of the 14 rows previously described. During the scanning step (or one of the re-scanning steps as the case may be), the three points that make up segment 41 are scanned determine the activity readings associated with those particular subcomponents of the array. The readings are represented by dashed box 42, where it can be seen that the activity readings for the segment are, from left to right, 24, 25, and 36. In one or more of the treatment steps, the right-most point 44 in the segment 41 (the point having a value of 36) is treated. The segment points that have been treated (point 44 in this example) are re-scanned to determine new activity readings. In this example, the new value is shown as 49, which is the secondary value associated with point 44 and shown in dashed box 43. It should be understood that the activity readings may be in any suitable units, or without units.

FIG. 5 illustrates scanning and measuring activity reading values for three points associated with a second segment 51. Dashed box 52 illustrates the values associated with this assessment. It can be seen that the middle value is highest. Thus, a point-stimulation (i.e., treatment) is applied to that point in the segment. The new activity reading for that point is 54 and is shown in dashed box 53.

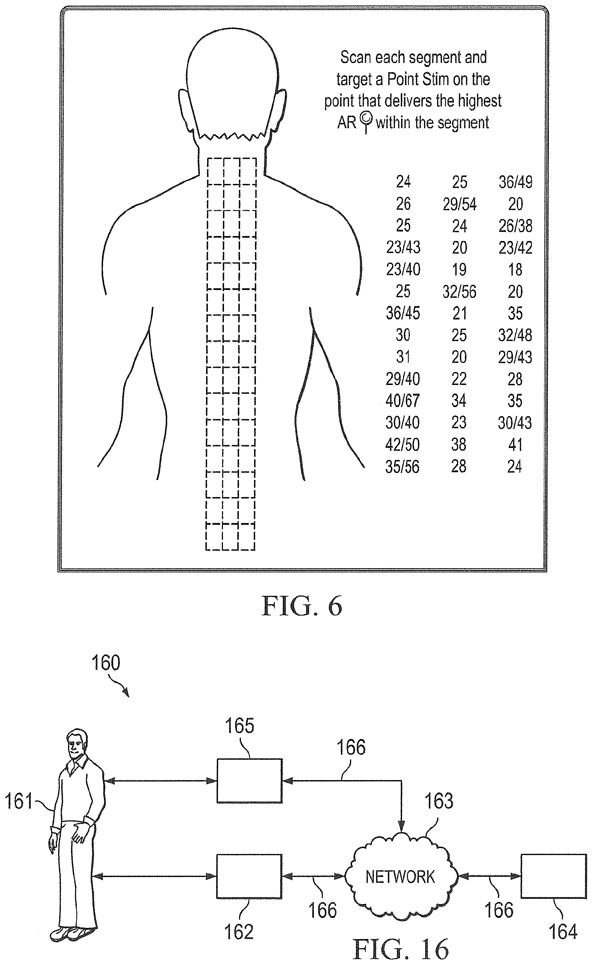

FIG. 6 illustrates the entire treatment area array after activity readings have been measured for each point of each segment, the highest-value points in each segment have received a point-stimulation treatment, and new activity readings have been determined for the points that received point-stimulation.

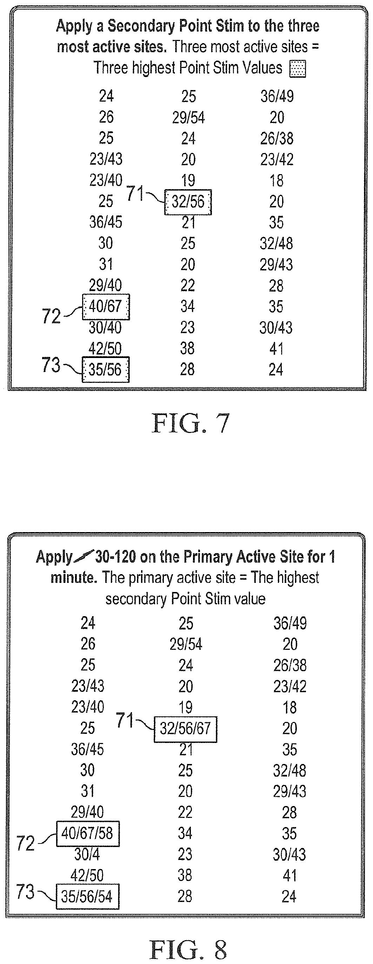

FIG. 7 illustrates another aspect that may be incorporated into one or more protocols described herein. At this point in the process, the three most active sites in the array are determined. It can be seen that the second values of sites 71, 72, and 73 are 56, 67, and 56 respectively, and that these are the three highest values among the sites of the array. It should be understood that the selection of three most-active sites is an example, and a larger or smaller number of most-active sites may be determined. This figure also illustrates the concept of applying a second point-stimulation to the three most-active sites.

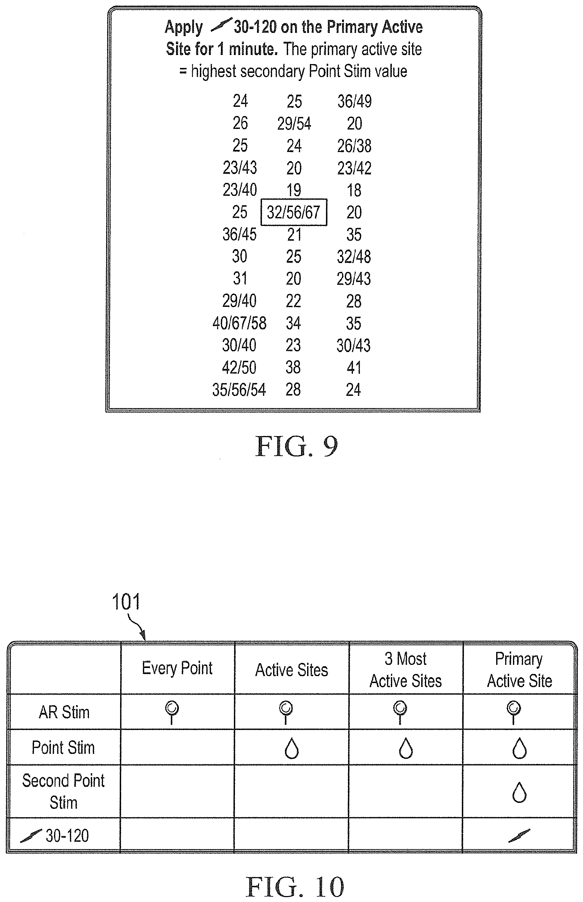

FIG. 8 illustrates the follow-on assessment of the three sites in FIG. 7 after the application of the second point-stimulation. The respective values are 67, 58 and 54. This figure also illustrates the concept of determining the single most-active site (e.g., the site having the highest activity reading), which is site 71. FIGS. 8 and 9 collectively illustrate treating this site with treatment energy. In at least one example embodiment the treatment energy is delivered for about one minute. Preferably, the treatment energy is at an energy level, wave form, frequency, etc. predetermined to achieve a desired treatment effect.

In at least certain embodiments, "activity reading" refers to an objective measurement of the effect that skin impedance has on a waveform of energy applied to that point. The activity reading may be used to provide a relative measurement of tissue impedance between distinct points on the skin. A higher activity reading may be used to indicate where treatment is to be applied. "Point stimulation" or "point stim" refers to treatment of a specific area of pain and/or low impedance. When a point-stimulation is completed, a "point stimulation value" may be assessed. In certain cases, a site with a relatively higher activity reading indicates a relatively-lower skin impedance. Also, in certain cases, a point-stim value is indicated of a secondary activity reading. The above description also reflects the concept of "multi-stim," which refers to a series of point-stims delivered to one treatment point.

FIG. 10 illustrates a treatment protocol execution grid 101. Grid 101 reflects the treatment area array assessment process of at least one example embodiment. A first assessment reading is done for all sites in the array. This is reflected in the upper left-hand box. It can be seen that a point-stimulation is applied to the most active sites (e.g., having the highest activity reading value) in each segment of the array. Then these sites are re-assessed. Then the three most-active sites in the entire array are determined and receive a second point-stimulation. Then, these three points are re-assessed. Then the most active site is determined and treated.

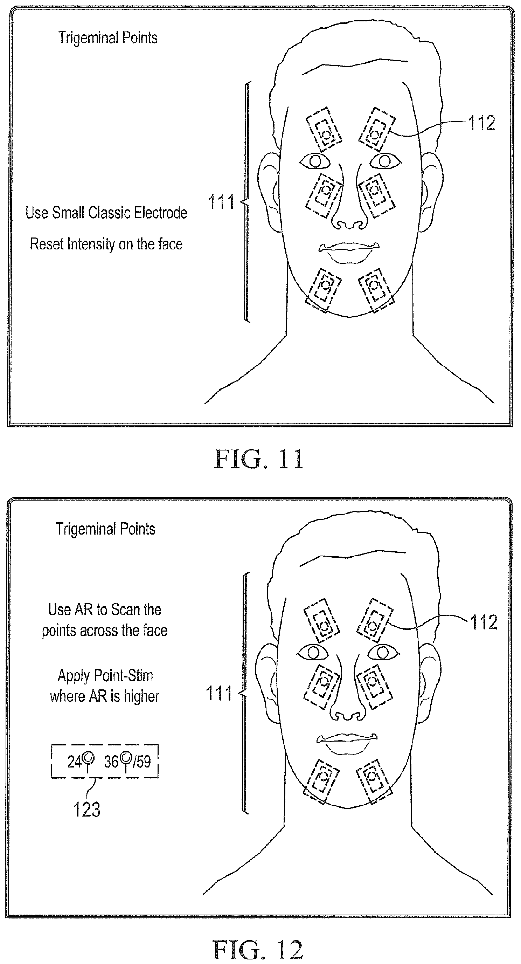

FIG. 11 illustrates assessment and treatment of a facial region. As previously discussed, this protocol may be useful for pain perceived in the facial region. But, it may also be useful for pain perceived in other regions, such as the back (for example). The sites 112 selected in the illustrated example may be referred to as trigeminal points. Sites 112 make up a treatment area array 111.

FIG. 12 illustrates a process similar to that already described in which array segments are scanned to elicit an activity reading (AR), which is a measure of the relative impedance of the respective sites. Thus, dashed box 123 represents activity readings for the lower segment of the array with respective values (left to right) of 24 and 36 Ohms. The value of 59 Ohms reflects the secondary reading of the right-most site after a point-stimulation is applied. Similar to the protocols already described, a point-stimulation was applied to the right-most site because this site had the highest activity reading of the two sites in the segment.

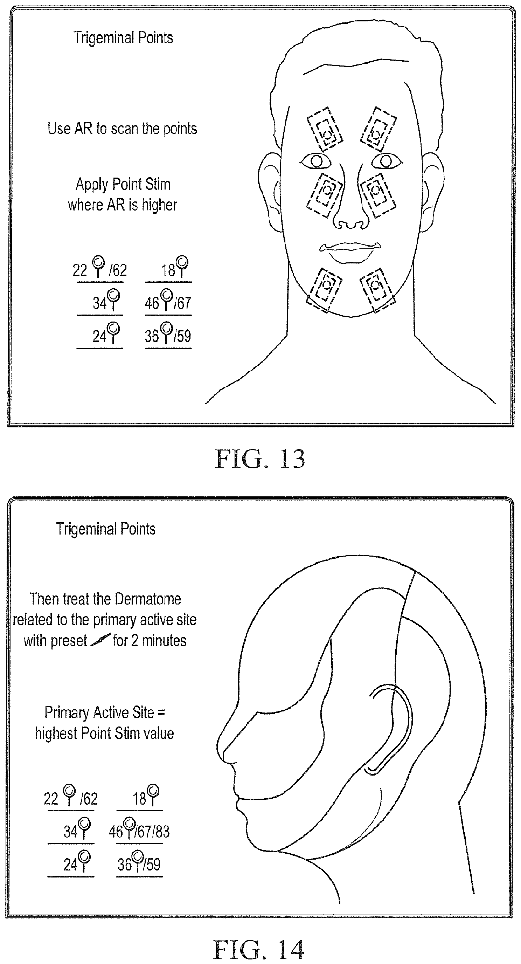

FIG. 13 illustrates the results of scanning the remaining segments, and rescanning those sites within each segment after point-stimulation is delivered to the respective sites having the highest activity reading within each segment. FIG. 14 illustrates the identification of the site within the array having the highest activity reading, as well as the treatment of that site. Treatment energy is reflected as a preset energy level delivered for two minutes. The preset amount may be any appropriate energy level suitable for the treatment objectives. The time of delivery of the treatment signal may also be varied to suit treatment objectives.

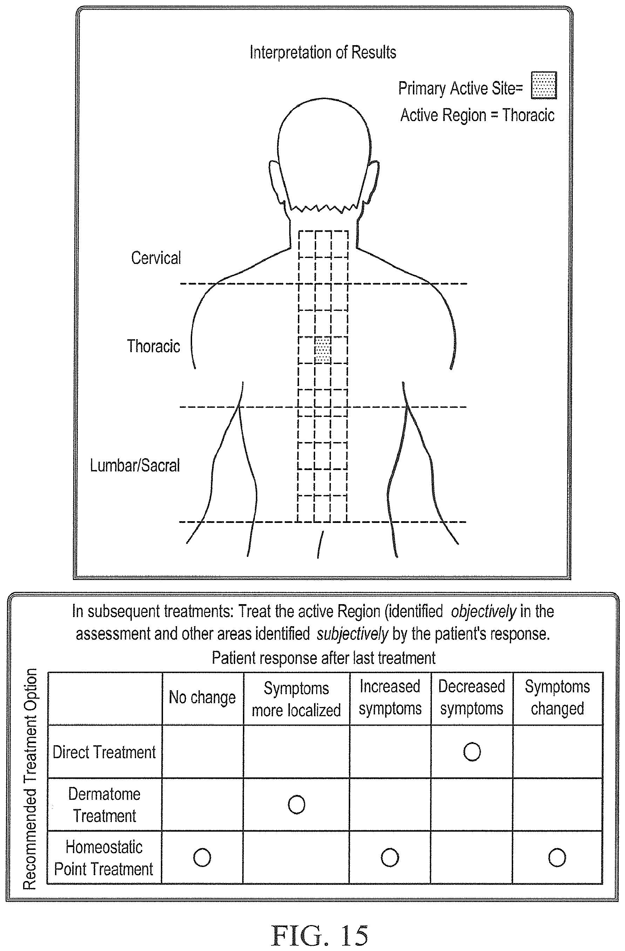

FIG. 15 illustrates interpretation of the results of assessment, activity reading, point-stimulation, and/or treatment. In this example, a primary site has been identified in the Thoracic region of the back.

FIG. 15 also illustrates a chart which may be used to track a treatment protocol. The chart reflects treatment of objectively identified areas and subjectively identified areas. Objectively identified areas include those areas identified during the assessment protocols as already described. Subjectively identified areas include those areas identified by a patient's response to application of neurostimulation energy including point-stimulation and/or treatment.

FIG. 16 illustrates an example system 160 for executing one or more of the protocols described elsewhere herein. System 160 includes a user 161, an assessment apparatus 162, and a computing platform 164 connected to each other by a network 163. Although the figure illustrates a particular arrangement of user 161, assessment apparatus 162, treatment apparatus 165, computing platform 164, and network 163, this disclosure contemplates any suitable arrangement of these elements. Moreover, although the figure illustrates a particular number of each element, this disclosure contemplates any suitable number of elements.

In particular embodiments, computing platform 164 may be a network-addressable computing system that can host various applications, such as, for example, assessment and treatment applications. Computing platform 164 may generate, store, receive, and transmit information, such as, for example, activity readings and or relative impedance measurements, treatment area array configuration data, array site information, point-stimulation delivery, and treatment energy delivery. Computing platform 164 may be accessed by the other components of system 160 either directly or via network 163.

This disclosure contemplates any suitable network 163. As an example and not by way of limitation, one or more portions of network 163 may include an ad hoc network, an intranet, an extranet, a virtual private network (VPN), a local area network (LAN), a wireless LAN (WLAN), a wide area network (WAN), a wireless WAN (WW AN), a metropolitan area network (MAN), a portion of the Internet, a portion of the Public Switched Telephone Network (PSTN), a cellular telephone network, or a combination of two or more of these. Network 163 may include one or more networks 163.

Connections 166 may connect assessment apparatus 162, treatment apparatus 165, and computing platform 164 to network 163 and/or to each other. This disclosure contemplates any suitable connections 166. In particular embodiments, one or more connections 166 include one or more wireline (such as for example Digital Subscriber Line (DSL) or Data Over Cable Service Interface Specification (DOCSIS)), wireless (such as for example Wi-Fi or Worldwide Interoperability for Microwave Access (WiMAX)) or optical (such as for example Synchronous Optical Network (SONET) or Synchronous Digital Hierarchy (SDH)) connections. In particular embodiments, one or more connections 166 each include an ad hoc network, an intranet, an extranet, a VPN, a LAN, a WLAN, a WAN, a WWAN, a MAN, a portion of the Internet, a portion of the PSTN, a cellular telephone network, another connection 166, or a combination of two or more such connections 166. Connections 166 need not necessarily be the same throughout system 160. One or more first connections 166 may differ in one or more respects from one or more second connections 166.

Various components such as, for example, assessment apparatus 162 and treatment apparatus 165, may include computers, printers, a displays, user interfaces, and network interfaces (not expressly shown).

Printers may include any suitable type of printer, such as, for example, a dot-matrix printer, an ink-jet printer, a solid ink printer, a dye-sublimation printer, a laser printer, a thermal printer, another suitable printer, or two or more such printers. The printer may print on any suitable media, such as, for example, sheet paper, printer paper, plotter paper, photo paper, paper rolls, printer label rolls, label sheets, boxes, parcels, another suitable media, or two or more such media. As an example and not by way of limitation, the printer may be used to print postage indicia (e.g., postage stamps). As another example and not by way of limitation, the printer may be used as a label printer. As yet another example and not by way of limitation, the printer may be used as a standard printer. The printer may be operable to print any of the charts or other images referenced herein.

Network interface may include any suitable type of network interface, such as, for example wireless or Ethernet based. Any of the components may use the network interface to connect with other components or systems via any suitable connection, such as, for example, a connection 166.

In particular embodiments, various components of system 160 may be powered by any suitable power source. These may include, for example, an external source (such as, for example, an AC or DC power outlet) or an internal source (such as, for example, rechargeable or non-rechargeable batteries).

In particular embodiments, various system components may include one or more displays. The display may render, visualize, display, message, and publish to one or more users based on input to, or output from, any of the various components. Input and output can be transmitted to the display by any suitable connection. The display can include any suitable I/O device that can enable communication between a user and display. As an example and not by way of limitation, the display can include a video monitor, speaker, touchscreen, printer, another suitable I/O device, or a combination of two or more of these.

In particular embodiments, various system components may receive user-input from one or more users via the display. As an example and not by way of limitation, the display may be a type of touchscreen, such as a resistive touchscreen, a capacitive touchscreen, an infrared touchscreen, or another suitable type of touchscreen. The user may click, touch, or otherwise interact with the display to select and input commands and to perform other actions. As an example and not by way of limitation, components may receive user-input via keyboard, keypad, microphone, monitor, mouse, printer, scale, scanner, speaker, still camera, stylus, tablet, trackball, video camera, another suitable I/O device, or a combination of two or more of these. As another example, and not by way of limitation, components may receive user input via a peripheral I/O device, such as a detachable keyboard, keypad, microphone, monitor, mouse, printer, scale, scanner, speaker, still camera, stylus, tablet, trackball, video camera, another suitable I/O device, or a combination of two or more of these.

In particular embodiments, various system components may receive user-input from one or more users via a user interface that is displayed on a display. As an example and not by way of limitation, the user interface may be a graphic user interface that allows one or more users to interact with the respective system component. A user may click, touch, or otherwise interact with the user interface to provide input to the respective system component. The user interface may be generated by any suitable program or application. As an example and not by way of limitation, the user interface may be provided in a structured document and processed by a browser client of the respective system component. A user can use the browser client or other application to access a user interface over a network (such as, for example, network 163). The user interface may be automatically generated and presented to the user in response to the user accessing one of the system components, a third-party website, or executing an application on one of the system components. As another example, and not by way of limitation, the user interface may be provided by a dedicated client application hosted on the respective system component.

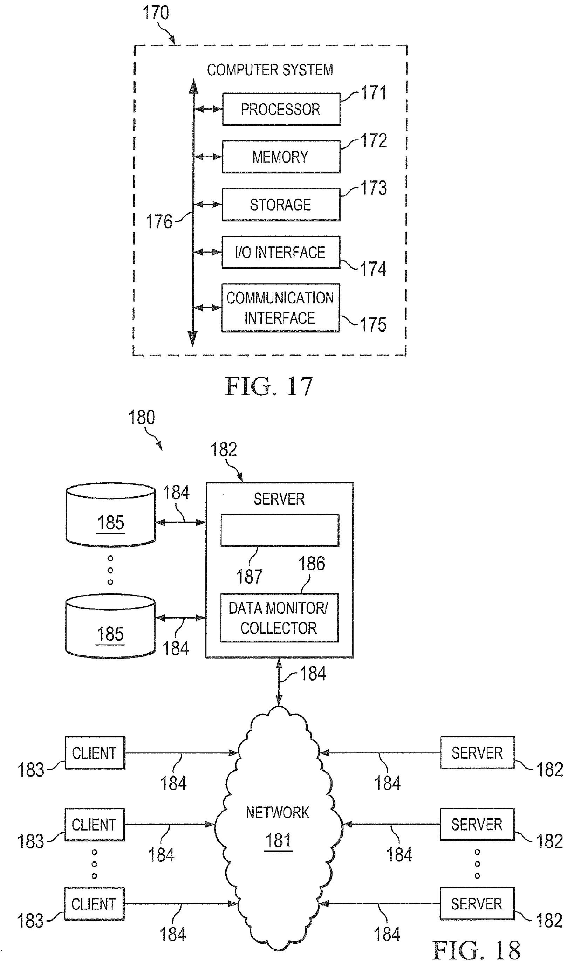

FIG. 17 illustrates an example computer system 170. In particular embodiments, one or more computer systems 170 perform one or more steps of one or more methods described or illustrated herein. In particular embodiments, one or more computer systems 170 provide functionality described or illustrated herein. In particular embodiments, software running on one or more computer systems 170 performs one or more steps of one or more methods described or illustrated herein or provides functionality described or illustrated herein. Particular embodiments include one or more portions of one or more computer systems 170.

This disclosure contemplates any suitable number of computer systems 170. This disclosure contemplates computer system 170 taking any suitable physical form. As example, and not by way of limitation, computer system 170 may be an embedded computer system, a system-on-chip (SOC), a single-board computer system (SBC) (such as, for example, a computer-on-module (COM) or system-on-module (SON)), a desktop computer system, a laptop or notebook computer system, an interactive kiosk, a mainframe, a mesh of computer systems, a mobile telephone, a personal digital assistant (PDA), a server, a tablet computer system, or a combination of two or more of these. Where appropriate, computer system 170 may include one or more computer systems 170; be unitary or distributed; span multiple locations; span multiple machines; span multiple data centers; or reside in a cloud, which may include one or more cloud components in one or more networks. Where appropriate, one or more computer systems 170 may perform without substantial spatial or temporal limitation one or more steps of one or more methods described or illustrated herein. As an example, and not by way of limitation, one or more computer systems 170 may perform in real time or in batch mode one or more steps of one or more methods described or illustrated herein. One or more computer systems 170 may perform at different times or at different locations one or more steps of one or more methods described or illustrated herein, where appropriate.

In particular embodiments, computer system 170 includes a processor 171, memory 172, storage 173, an input/output (I/O) interface 174, a communication interface 175, and a bus 176. Although this disclosure describes and illustrates a particular computer system having a particular number of particular components in a particular arrangement, this disclosure contemplates any suitable computer system having any suitable number of any suitable components in any suitable arrangement.

In particular embodiments, processor 171 includes hardware for executing instructions, such as those making up a computer program. As an example and not by way of limitation, to execute instructions, processor 171 may retrieve (or fetch) the instructions from an internal register, an internal cache, memory 172, or storage 173; decode and execute them; and then write one or more results to an internal register, an internal cache, memory 172, or storage 173. In particular embodiments, processor 171 may include one or more internal caches for data, instructions, or addresses. This disclosure contemplates processor 171 including any suitable number of any suitable internal caches, where appropriate. As an example, and not by way of limitation, processor 171 may include one or more instruction caches, one or more data caches, and one or more translation lookaside buffers (TLBs). Instructions in the instruction caches may be copies of instructions in memory 172 or storage 173, and the instruction caches may speed up retrieval of those instructions by processor 171. Data in the data caches may be copies of data in memory 172 or storage 173 for instructions executing at processor 171 to operate on the results of previous instructions executed at processor 171 for access by subsequent instructions executing at processor 171 or for writing to memory 172 or storage 173; or other suitable data. The data caches may speed up read or write operations by processor 171. The TLBs may speed up virtual-address translation for processor 171. In particular embodiments, processor 171 may include one or more internal registers for data, instructions, or addresses. This disclosure contemplates processor 171 including any suitable number of any suitable internal registers, where appropriate. Where appropriate, processor 171 may include one or more arithmetic logic units (ALUs); be a multicore processor; or include one or more processors 171. Although this disclosure describes and illustrates a particular processor, this disclosure contemplates any suitable processor.

In particular embodiments, memory 172 includes memory for storing instructions for processor 171 to execute or data for processor 171 to operate on. As an example, and not by way of limitation, computer system 170 may load instructions from storage 173 or another source (such as, for example, another computer system 170) to memory 172. Processor 171 may then load the instructions from memory 172 to an internal register or internal cache. To execute the instructions, processor 171 may retrieve the instructions from the internal register or internal cache and decode them. During or after execution of the instructions, processor 171 may write one or more results (which may be intermediate or final results) to the internal register or internal cache. Processor 171 may then write one or more of those results to memory 172. In particular embodiments, processor 171 executes only instructions in one or more internal registers or internal caches or in memory 172 (as opposed to storage 173 or elsewhere) and operates only on data in one or more internal registers or internal caches or in memory 172 (as opposed to storage 173 or elsewhere). One or more memory buses (which may each include an address bus and a data bus) may couple processor 171 to memory 172. Bus 176 may include one or more memory buses, as described below. In particular embodiments, one or more memory management units (MMUs) reside between processor 171 and memory 172 and facilitate accesses to memory 172 requested by processor 171. In particular embodiments, memory 172 includes random access memory (RAM). This RAM may be volatile memory, where appropriate. Where appropriate, this RAM may be dynamic RAM (DRAM) or static RAM (SRAM). Moreover, where appropriate, this RAM may be single-ported or multi-ported RAM. This disclosure contemplates any suitable RAM. Memory 172 may include one or more memories 172, where appropriate. Although this disclosure describes and illustrates particular memory, this disclosure contemplates any suitable memory.

In particular embodiments, storage 173 includes mass storage for data or instructions. As an example and not by way of limitation, storage 173 may include an HDD, a floppy disk drive, flash memory, an optical disc, a magneto-optical disc, magnetic tape, or a Universal Serial Bus (USB) drive or a combination of two or more of these. Storage 173 may include removable or non-removable (or fixed) media, where appropriate. Storage 173 may be internal or external to computer system 170, where appropriate. In particular embodiments, storage 173 is non-volatile, solid-state memory. In particular embodiments, storage 173 includes read-only memory (ROM). Where appropriate, this ROM may be mask-programmed ROM, programmable ROM (PROM), erasable PROM (EPROM), electrically erasable PROM (EEPROM), electrically alterable ROM (BARON), or flash memory or a combination of two or more of these. This disclosure contemplates mass storage 173 taking any suitable physical form. Storage 173 may include one or more storage control units facilitating communication between processor 171 and storage 173, where appropriate. Where appropriate, storage 173 may include one or more storages 173. Although this disclosure describes and illustrates particular storage, this disclosure contemplates any suitable storage.

In particular embodiments, I/O interface 174 includes hardware, software, or both providing one or more interfaces for communication between computer system 170 and one or more I/O devices. Computer system 170 may include one or more of these I/O devices, where appropriate. One or more of these I/O devices may enable communication between a person and computer system 170. As an example, and not by way of limitation, an I/O device may include a keyboard, keypad, microphone, monitor, mouse, printer, scanner, speaker, still camera, stylus, tablet, touch screen, trackball, video camera, another suitable I/O device or a combination of two or more of these. An I/O device may include one or more sensors. This disclosure contemplates any suitable I/O devices and any suitable I/O interfaces 908 for them. Where appropriate, I/O interface 174 may include one or more device or software drivers enabling processor 171 to drive one or more of these I/O devices. I/O interface 174 may include one or more I/O interfaces 174, where appropriate. Although this disclosure describes and illustrates a particular I/O interface, this disclosure contemplates any suitable I/O interface.

In particular embodiments, communication interface 175 includes hardware, software, or both providing one or more interfaces for communication (such as, for example, packet-based communication) between computer system 170 and one or more other computer systems 170 or one or more networks. As an example and not by way of limitation, communication interface 175 may include a network interface controller (NIC) or network adapter for communicating with an Ethernet or other wire-based network or a wireless NIC (WNIC) or wireless adapter for communicating with a wireless network, such as a WI-FI network. This disclosure contemplates any suitable network and any suitable communication interface 175 for it. As an example and not by way of limitation, computer system 170 may communicate with an ad hoc network, a personal area network (PAN), a local area network (LAN), a wide area network (WAN), a metropolitan area network (MAN), or one or more portions of the Internet or a combination of two or more of these. One or more portions of one or more of these networks may be wired or wireless. As an example, computer system 170 may communicate with a wireless PAN (WPAN) (such as, for example, a BLUETOOTH WPAN), a WI-FI network, a WIMAX network, a cellular telephone network (such as, for example, a Global System for Mobile Communications (GSM) network), or other suitable wireless network or a combination of two or more of these. Computer system 170 may include any suitable communication interface 175 for any of these networks, where appropriate. Communication interface 175 may include one or more communication interfaces 175, where appropriate. Although this disclosure describes and illustrates a particular communication interface, this disclosure contemplates any suitable communication interface.

In particular embodiments, bus 176 includes hardware, software, or both coupling components of computer system 170 to each other. As an example, and not by way of limitation, bus 176 may include an Accelerated Graphics Port (AGP) or other graphics bus, an Enhanced Industry Standard Architecture (EISA) bus, a front-side bus (FSB), a HYPERTRANSPORT (HT) interconnect, an Industry Standard Architecture (ISA) bus, an INFINIBAND interconnect, a low-pin-count (LPC) bus, a memory bus, a Micro Channel Architecture (MCA) bus, a Peripheral Component Interconnect (PCI) bus, a PCI-Express (PCI-X) bus, a serial advanced technology attachment (SATA) bus, a Video Electronics Standards Association local (VLB) bus, or another suitable bus or a combination of two or more of these. Bus 176 may include one or more buses 176, where appropriate. Although this disclosure describes and illustrates a particular bus, this disclosure contemplates any suitable bus or interconnect.

Herein, reference to a computer-readable storage medium encompasses one or more non-transitory, tangible computer-readable storage media possessing structure. As an example and not by way of limitation, a computer-readable storage medium may include a semiconductor-based or other integrated circuit (IC) (such, as for example, a field-programmable gate array (FPGA) or an application-specific IC (ASIC)), a hard disk, an HDD, a hybrid hard drive (HHD), an optical disc, an optical disc drive (ODD), a magneto-optical disc, a magneto-optical drive, a floppy disk, a floppy disk drive (FDD), magnetic tape, a holographic storage medium, a solid-state drive (SSD), a RAM-drive, a SECURE DIGITAL card, a SECURE DIGITAL drive, or another suitable computer-readable storage medium or a combination of two or more of these, where appropriate. Herein, reference to a computer-readable storage medium excludes any medium that is not eligible for patent protection under 35 U.S.C. §101. Herein, reference to a computer-readable storage medium excludes transitory forms of signal transmission (such as a propagating electrical or electromagnetic signal per se) to the extent that they are not eligible for patent protection under 35 U.S.C. §101. A computer-readable non-transitory storage medium may be volatile, non-volatile, or a combination of volatile and non-volatile, where appropriate.

This disclosure contemplates one or more computer-readable storage media implementing any suitable storage. In particular embodiments, a computer-readable storage medium implements one or more portions of processor 171 (such as, for example, one or more internal registers or caches), one or more portions of memory 172, one or more portions of storage 173, or a combination of these, where appropriate. In particular embodiments, a computer-readable storage medium implements RAM or ROM. In particular embodiments, a computer-readable storage medium implements volatile or persistent memory. In particular embodiments, one or more computer-readable storage media embody software. Herein, reference to software may encompass one or more applications, bytecode, one or more computer programs, one or more executables, one or more instructions, logic, machine code, one or more scripts, or source code, and vice versa, where appropriate. In particular embodiments, software includes one or more application programming interfaces (APIs). This disclosure contemplates any suitable software written or otherwise expressed in any suitable programming language or combination of programming languages. In particular embodiments, software is expressed as source code or object code. In particular embodiments, software is expressed in a higher-level programming language, such as, for example, C, Perl, or a suitable extension thereof. In particular embodiments, software is expressed in a lower-level programming language, such as assembly language (or machine code). In particular embodiments, software is expressed in JA VA. In particular embodiments, software is expressed in Hyper Text Markup Language (HTML), Extensible Markup Language (XML), or other suitable markup language.

FIG. 18 illustrates an example network environment 180. This disclosure contemplates any suitable network environment 180. As an example, and not by way of limitation, although this disclosure describes and illustrates a network environment 180 that implements a client-server model, this disclosure contemplates one or more portions of a network environment 180 being peer-to-peer, where appropriate. Particular embodiments may operate in whole or in part in one or more network environments 180. In particular embodiments, one or more elements of network environment 180 provide functionality described or illustrated herein. Particular embodiments include one or more portions of network environment 180. Network environment 180 includes a network 181 coupling one or more servers 182 and one or more clients 183 to each other. This disclosure contemplates any suitable network 1010. As an example, and not by way of limitation, one or more portions of network 181 may include an ad hoc network, an intranet, an extranet, a virtual private network (VPN), a local area network (LAN), a wireless LAN (WLAN), a wide area network (WAN), a wireless WAN (WW AN), a metropolitan area network (MAN), a portion of the Internet, a portion of the Public Switched Telephone Network (PSTN), a cellular telephone network, or a combination of two or more of these. Network 181 may include one or more networks 181.

Links 184 couple servers 182 and clients 183 to network 181 or to each other. This disclosure contemplates any suitable links 184. As an example, and not by way of limitation, one or more links 184 each include one or more wireline (such as, for example, Digital Subscriber Line (DSL) or Data Over Cable Service Interface Specification (DOCSIS>>, wireless (such as, for example, Wi-Fi or Worldwide Interoperability for Microwave Access (WiMAX) or optical (such as, for example, Synchronous Optical Network (SONET) or Synchronous Digital Hierarchy (SDH)) links 184. In particular embodiments, one or more links 184 each includes an intranet, an extranet, a VPN, a LAN, a WLAN, a WAN, a MAN, a communications network, a satellite network, a portion of the Internet, or another link 184 or a combination of two or more such links 184. Links 184 need not necessarily be the same throughout network environment 180. One or more first links 184 may differ in one or more respects from one or more second links 184.

This disclosure contemplates any suitable servers 182. As an example, and not by way of limitation, one or more servers 182 may each include one or more applications servers, catalog servers, communications servers, database servers, exchange servers, fax servers, file servers, home servers, mail servers, message servers, name or DNS servers, print servers, proxy servers, sound servers, standalone servers, web servers, or web-feed servers. In particular embodiments, a server 182 includes hardware, software, or both for providing the functionality of server 182. As an example, and not by way of limitation, a server 182 that operates as a web server may be capable of hosting websites containing web pages or elements of web pages and include appropriate hardware, software, or both for doing so. In particular embodiments, a web server may host HTML or other suitable files or dynamically create or constitute files for web pages on request. In response to a Hyper Text Transfer Protocol (HTTP) or other request from a client 183, the web server may communicate one or more such files to client 183. As another example, a server 18 that operates as a database server may be capable of providing an interface for interacting with one or more data stores (such as, for example, data stores 185 described below). Where appropriate, a server 182 may include one or more servers 182; be unitary or distributed; span multiple locations; span multiple machines; span multiple datacenters; or reside in a cloud, which may include one or more cloud components in one or more networks.

In particular embodiments, one or more links 184 may couple a server 182 to one or more data stores 185. A data store 185 may store any suitable information, and the contents of a data store 185 may be organized in any suitable manner. As an example, and not by way or limitation, the contents of a data store 185 may be stored as a dimensional, flat, hierarchical, network, object-oriented, relational, XML, or other suitable database or a combination or two or more of these. A data store 185 (or a server 182 coupled to it) may include a database-management system or other hardware or software for managing the contents of data store 185. The database-management system may perform read and write operations, delete or erase data, perform data de-duplication, query or search the contents of data store 185, or provide other access to data store 185.

In particular embodiments, one or more servers 182 may each include one or more data monitors/collectors 186. A data monitor/collection 186 may include hardware, software, or both for providing the functionality of data collector/collector 186. As an example, and not by way of limitation, a data monitor/collector 186 at a server 182 may monitor and collect network-traffic data at server 182 and store the network-traffic data in one or more data stores 185. In particular embodiments, server 182 or another device may extract pairs of search queries and selected URLs from the network-traffic data, where appropriate. In at least one embodiment, a data monitor/collector receives data from an assessment apparatus. The data may include any of the assessment/treatment information described herein or any derivations or modifications of such data. Such data may include, for example, activity readings and/or relative impedance measurements.

One or more servers 182 may include, or otherwise function as, one or more computing platforms 187. In at least one embodiment, at least one computing platform 187 receives date from data monitor/collector 186. Platform 187 includes a number of modules, which collectively provide the functionality described herein, such as determining site values for point-stimulation and treatment, as well as determining and executing the steps of the various protocols.

This disclosure contemplates any suitable clients 183. A client 183 may enable a user at client 183 to access or otherwise communicate with network 181, servers 182, or other clients 183. As an example, and not by way of limitation, a client 183 may have a web browser, such as MICROSOFT INTERNET EXPLORER or MOZILLA FIREFOX, and may have one or more add-ons, plug-ins, or other extensions, such as GOOGLE TOOLBAR or YAHOO TOOLBAR. A client 183 may be an electronic device including hardware, software, or both for providing the functionality of client 183. As an example, and not by way of limitation, a client 183 may, where appropriate, be an embedded computer system, an SOC, an SBC (such as, for example, a COM or SOM), a desktop computer system, a laptop or notebook computer system, an interactive kiosk, a mainframe, a mesh of computer systems, a mobile telephone, a PDA, a netbook computer system, a server, a tablet computer system, or a combination of two or more of these. Where appropriate, a client 183 may include one or more clients 183; be unitary or distributed; span multiple locations; span multiple machines; span multiple datacenters; or reside in a cloud, which may include one or more cloud components in one or more networks.

The invention is defined in the following claims, other embodiments and methods being merely exemplary.

## Claims

1. A system (160) of providing neurostimulation, the system (160) comprising:
- an assessment apparatus (162) operable to measure an activity reading value of each of a plurality of sites within a treatment area array;
- a computer (164) operable to receive each of the activity reading values from the assessment apparatus (162), and to determine one or more subsets of the plurality of sites for stimulation,
- the system (160) being adapted for conducting the following steps:
- establishing a treatment area array having a plurality of sites;
- scanning a plurality of sites of the treatment area array to determine an activity reading value for each of the plurality of sites of the treatment area array;
- determining a first subset of the plurality of sites of the treatment area array based on the activity reading values;
- stimulating each site within the first subset of the plurality of sites;
- re-scanning each site within the first subset of the plurality of sites to determine a second activity reading value for each of the plurality of the stimulated sites within the first subset;
- determining a second subset of the plurality of sites of the treatment area array wherein the second subset of sites is selected from the first subset of sites, and wherein the determining step is based on the second activity reading values obtained after re-scanning the first subset of sites;
- stimulating each site within the second subset of sites;
- re-scanning each site within the second subset of the plurality of sites to determine a third activity reading value for each of the plurality of the stimulated sites within the second subset;
- determining a third subset of the plurality of sites of the treatment area array wherein the third subset of sites is selected from the second subset of sites, and wherein the determining step is based on the third activity reading values obtained after re-scanning the second subset of sites.

2. The system (160) of claim 1, further adapted for repeating at least one of the determining, stimulating, and re-scanning steps to identify a subset of sites for treatment.

3. The system (160) of claim 1, wherein the determining step for determining a subset comprises determining a subset as a pre-established percentage of the plurality of sites.

4. The system (160) of claim 3, wherein a subset comprises a pre-established percentage of the plurality of sites having the highest activity readings.

5. The system (160) of claim 1, wherein the determining step for determining a subset comprises determining a subset as a predetermined number of the plurality of sites.

6. The system (160) of claim 1, wherein the determining step for determining a subset is accomplished, at least partially, by selecting sites from a plurality of sites according to the position within the treatment area array.

7. The system (160) of claim 1, wherein the determining step for determining a subset comprises assigning a weighting value to at least one of a plurality of sites prior to determining the subset.

8. The system (160) of claim 1, wherein the stimulating step comprises stimulating one or more of the sites of a subset sequentially according to a position within the treatment area array.

9. The system (160) of claim 1, wherein the stimulating step comprises stimulating one or more of the sites of a subset sequentially according to an activity reading value.

10. The system (160) of claim 1, wherein the stimulating step comprises stimulating two or more of the sites of a subset simultaneously.

11. The system (160) of claim 1, further adapted for dividing the treatment area array into one or more segments, and wherein the determining step for determining a subset comprises determining at least one site within the segment to be included in the subset.

12. The system (160) of claim 1, wherein the assessment apparatus (162) is further operable to re-scan the first subset to determine a second activity reading for at least one site among the first subset of sites.

13. The system (160) of claim 1, wherein the computer (164) is operable to receive the second activity readings, and to determine a second subset of the first subset for stimulation.

14. The system (160) of claim 1, further comprising a non-transitory computer-readable medium, comprising software executable by a processor, and operable:
- to receive activity reading values from an assessment apparatus (162), and to determine one or more subsets of a plurality of sites of a treatment area array;
- the software being adapted for conducting the following steps:
- establishing a treatment area array having a plurality of sites;
- scanning a plurality of sites of the treatment area array to determine an activity reading value for each of the plurality of sites of the treatment area array;
- determining a first subset of the plurality of sites of the treatment area array based on the activity reading values;
- stimulating each site within the first subset of the plurality of sites;
- re-scanning each site within the first subset of the plurality of sites to determine a second activity reading value for each of the plurality of the stimulated sites within the first subset;
- determining a second subset of the plurality of sites of the treatment area array wherein the second subset of sites is selected from the first subset of sites, and wherein the determining step is based on the second activity reading values obtained after re-scanning the first subset of sites;
- stimulating each site within the second subset of sites;
- re-scanning each site within the second subset of the plurality of sites to determine a third activity reading value for each of the plurality of the stimulated sites within the second subset;
- determining a third subset of the plurality of sites of the treatment area array wherein the third subset of sites is selected from the second subset of sites, and wherein the determining step is based on the third activity reading values obtained after re-scanning the second subset of sites.

## Patentansprüche

1. System (160) zum Bewirken von Neurostimulation, wobei das System (160) aufweist:
- eine Bewertungsvorrichtung (162), die betrieben werden kann, um einen Aktivitätsmesswert von jeder von einer Mehrzahl von Stellen innerhalb eines Behandlungsgebiet-Arrays zu messen;
- einen Computer (164), der betrieben werden kann, um von der Bewertungsvorrichtung (162) jeden der Aktivitätsmesswerte zu empfangen, und um eine oder mehrere Teilmengen der Mehrzahl von Stellen zur Stimulation zu bestimmen,
- wobei das System (160) ausgestaltet ist, um die folgenden Schritte durchzuführen:
- Einrichten eines Behandlungsgebiet-Arrays, das eine Mehrzahl von Stellen beinhaltet;
- Scannen einer Mehrzahl von Stellen des Behandlungsgebiet-Arrays, um für jede der Mehrzahl von Stellen des Behandlungsgebiet-Arrays einen Aktivitätsmesswert zu bestimmen;
- Bestimmen einer ersten Teilmenge der Mehrzahl von Stellen des Behandlungsgebiet-Arrays auf Basis der Aktivitätsmesswerte;
- Stimulieren von jeder Stelle innerhalb der ersten Teilmenge der Mehrzahl von Stellen;
- erneutes Scannen von jeder Stelle innerhalb der ersten Teilmenge der Mehrzahl von Stellen, um für jede der Mehrzahl der stimulierten Stellen innerhalb der ersten Teilmenge einen zweiten Aktivitätsmesswert zu bestimmen;
- Bestimmen einer zweiten Teilmenge der Mehrzahl von Stellen des Behandlungsgebiet-Arrays, wobei die zweite Teilmenge von Stellen aus der ersten Teilmenge von Stellen ausgewählt wird, und wobei der Schritt des Bestimmens auf den zweiten Aktivitätsmesswerten basiert, die nach dem erneuten Scannen der ersten Teilmenge von Stellen erhalten werden;
- Stimulieren von jeder Stelle innerhalb der zweiten Teilmenge von Stellen;
- erneutes Scannen von jeder Stelle innerhalb der zweiten Teilmenge der Mehrzahl von Stellen, um für jede der Mehrzahl der stimulierten Stellen innerhalb der zweiten Teilmenge einen dritten Aktivitätsmesswert zu bestimmen;
- Bestimmen einer dritten Teilmenge der Mehrzahl von Stellen des Behandlungsgebiet-Arrays, wobei die dritte Teilmenge von Stellen aus der zweiten Teilmenge von Stellen ausgewählt wird, und wobei der Schritt des Bestimmens auf den dritten Aktivitätsmesswerten basiert, die nach dem erneuten Scannen der zweiten Teilmenge von Stellen erhalten werden.

2. System (160) nach Anspruch 1, das außerdem ausgestaltet ist, um zumindest einen der Schritte des Bestimmens, des Stimulierens und des erneuten Scannens zu wiederholen, um eine Teilmenge von Stellen zur Behandlung zu identifizieren.

3. System (160) nach Anspruch 1, wobei der Schritt des Bestimmens zum Bestimmen einer Teilmenge das Bestimmen einer Teilmenge als ein zuvor eingerichteter Prozentsatz der Mehrzahl von Stellen umfasst.

4. System (160) nach Anspruch 3, wobei eine Teilmenge einen zuvor eingerichteten Prozentsatz der Mehrzahl von Stellen enthält, die die höchsten Aktivitätsmesswerte haben.

5. System (160) nach Anspruch 1, wobei der Schritt des Bestimmens zum Bestimmen einer Teilmenge das Bestimmen einer Teilmenge als eine vorbestimmte Anzahl der Mehrzahl von Stellen umfasst.

6. System (160) nach Anspruch 1, wobei der Schritt des Bestimmens zum Bestimmen einer Teilmenge zumindest teilweise erreicht wird, indem Stellen aus einer Mehrzahl von Stellen ausgewählt werden, und zwar basierend auf der Position in dem Behandlungsgebiet-Array.

7. System (160) nach Anspruch 1, wobei der Schritt des Bestimmens zum Bestimmen einer Teilmenge das Zuweisen eines Gewichtungswertes zu mindestens einer der Mehrzahl von Stellen umfasst, und zwar vor dem Bestimmen der Teilmenge.

8. System (160) nach Anspruch 1, wobei der Schritt des Stimulierens das sequentielle Stimulieren von einer oder mehr der Stellen einer Teilmenge umfasst, und zwar basierend auf einer Position innerhalb des Behandlungsgebiet-Arrays.

9. System (160) nach Anspruch 1, wobei der Schritt des Stimulierens das sequentielle Stimulieren von einer oder mehr der Stellen einer Teilmenge umfasst, und zwar basierend auf einem Aktivitätsmesswert.

10. System (160) nach Anspruch 1, wobei der Schritt des Stimulierens das simultane Stimulieren von zwei oder mehr der Stellen einer Teilmenge umfasst.

11. System (160) nach Anspruch 1, das außerdem ausgestaltet ist, um das Behandlungsgebiet-Array in ein oder mehr Segmente zu unterteilen, und wobei der Schritt des Bestimmens zum Bestimmen einer Teilmenge das Bestimmen von mindestens einer Stelle innerhalb des Segments umfasst, um in der Teilmenge enthalten zu sein.

12. System (160) nach Anspruch 1, wobei die Bewertungsvorrichtung (162) außerdem betrieben werden kann, um die erste Teilmenge erneut zu scannen, um einen zweiten Aktivitätsmesswert für mindestens eine Stelle innerhalb der ersten Teilmenge von Stellen zu bestimmen.

13. System (160) nach Anspruch 1, wobei der Computer (164) betrieben werden kann, um die zweiten Aktivitätsmesswerte zu empfangen, und um eine zweite Teilmenge der ersten Teilmenge zur Stimulation zu bestimmen.

14. System (160) nach Anspruch 1, außerdem mit einem nicht-flüchtigen, computerlesbaren Medium, das Software enthält, die durch einen Prozessor ausgeführt werden kann, und betrieben werden kann:
- um Aktivitätsmesswerte von einer Bewertungsvorrichtung (162) zu empfangen, und um eine oder mehrere Teilmengen von einer Mehrzahl von Stellen eines Behandlungsgebiet-Arrays zu bestimmen;
- wobei die Software ausgestaltet ist, um die folgenden Schritte durchzuführen:
- Einrichten eines Behandlungsgebiet-Arrays, das eine Mehrzahl von Stellen beinhaltet;
- Scannen einer Mehrzahl von Stellen des Behandlungsgebiet-Arrays, um für jede der Mehrzahl von Stellen des Behandlungsgebiet-Arrays einen Aktivitätsmesswert zu bestimmen;
- Bestimmen einer ersten Teilmenge der Mehrzahl von Stellen des Behandlungsgebiet-Arrays auf Basis der Aktivitätsmesswerte;
- Stimulieren von jeder Stelle innerhalb der ersten Teilmenge der Mehrzahl von Stellen;
- erneutes Scannen von jeder Stelle innerhalb der ersten Teilmenge der Mehrzahl von Stellen, um für jede der Mehrzahl der stimulierten Stellen innerhalb der ersten Teilmenge einen zweiten Aktivitätsmesswert zu bestimmen;
- Bestimmen einer zweiten Teilmenge der Mehrzahl von Stellen des Behandlungsgebiet-Arrays, wobei die zweite Teilmenge von Stellen aus der ersten Teilmenge von Stellen ausgewählt wird, und wobei der Schritt des Bestimmens auf den zweiten Aktivitätsmesswerten basiert, die nach dem erneuten Scannen der ersten Teilmenge von Stellen erhalten werden;
- Stimulieren von jeder Stelle innerhalb der zweiten Teilmenge von Stellen;
- erneutes Scannen von jeder Stelle innerhalb der zweiten Teilmenge der Mehrzahl von Stellen, um für jede der Mehrzahl von stimulierten Stellen innerhalb der zweiten Teilmenge einen dritten Aktivitätsmesswert zu bestimmen;
- Bestimmen einer dritten Teilmenge der Mehrzahl von Stellen des Behandlungsgebiet-Arrays, wobei die dritte Teilmenge von Stellen aus der zweiten Teilmenge von Stellen ausgewählt wird, und wobei der Schritt des Bestimmens auf den dritten Aktivitätsmesswerten basiert, die nach dem erneuten Scannen der zweiten Teilmenge von Stellen erhalten werden.

## Revendications

1. Système (160) de fourniture de neurostimulation, le système (160) comprenant:
- un appareil d'évaluation (162) servant à mesurer une valeur de mesure d'activité de chaque site parmi une pluralité de sites au sein d'un groupement de zones de traitement;
- un ordinateur (164) servant à recevoir chacune des valeurs de mesure d'activité en provenance de l'appareil d'évaluation (162), et à déterminer un ou plusieurs sous-ensembles de la pluralité de sites pour une stimulation,
- le système (160) étant conçu pour effectuer les étapes suivantes:
- établissement d'un groupement de zones de traitement comportant une pluralité de sites;
- balayage d'une pluralité de sites du groupement de zones de traitement pour déterminer une valeur de mesure d'activité pour chaque site de la pluralité de sites du groupement de zones de traitement;
- détermination d'un premier sous-ensemble de la pluralité de sites du groupement de zones de traitement sur la base des valeurs de mesure d'activité;
- stimulation de chaque site au sein du premier sous-ensemble de la pluralité de sites;
- rebalayage de chaque site au sein du premier sous-ensemble de la pluralité de sites pour déterminer une deuxième valeur de mesure d'activité pour chaque site de la pluralité de sites stimulés au sein du premier sous-ensemble;
- détermination d'un deuxième sous-ensemble de la pluralité de sites du groupement de zones de traitement, le deuxième sous-ensemble de sites étant sélectionné dans le premier sous-ensemble de sites, et l'étape de détermination étant basée sur les deuxièmes valeurs de mesure d'activité obtenues après rebalayage du premier sous-ensemble de sites;
- stimulation de chaque site au sein du deuxième sous-ensemble de sites;
- rebalayage de chaque site au sein du deuxième sous-ensemble de la pluralité de sites pour déterminer une troisième valeur de mesure d'activité pour chaque site de la pluralité de sites stimulés au sein du deuxième sous-ensemble;
- détermination d'un troisième sous-ensemble de la pluralité de sites du groupement de zones de traitement, le troisième sous-ensemble de sites étant sélectionné dans le deuxième sous-ensemble de sites, et l'étape de détermination étant basée sur les troisièmes valeurs de mesure d'activité obtenues après rebalayage du deuxième sous-ensemble de sites.

2. Système (160) selon la revendication 1, conçu en outre pour répéter au moins une des étapes de détermination, de stimulation et de rebalayage pour identifier un sous-ensemble de sites pour un traitement.

3. Système (160) selon la revendication 1, dans lequel l'étape de détermination destinée à déterminer un sous-ensemble comprend la détermination d'un sous-ensemble sous la forme d'un pourcentage préétabli de la pluralité de sites.

4. Système (160) selon la revendication 3, dans lequel un sous-ensemble comprend un pourcentage préétabli de la pluralité de sites ayant les plus hautes mesures d'activité.

5. Système (160) selon la revendication 1, dans lequel l'étape de détermination destinée à déterminer un sous-ensemble comprend la détermination d'un sous-ensemble sous la forme d'un nombre prédéterminé de la pluralité de sites.

6. Système (160) selon la revendication 1, dans lequel l'étape de détermination destinée à déterminer un sous-ensemble est accomplie, au moins partiellement, par sélection de sites parmi une pluralité de sites en fonction de la position au sein du groupement de zones de traitement.

7. Système (160) selon la revendication 1, dans lequel l'étape de détermination destinée à déterminer un sous-ensemble comprend l'attribution d'une valeur de pondération à au moins un site d'une pluralité de sites préalablement à la détermination du sous-ensemble.

8. Système (160) selon la revendication 1, dans lequel l'étape de stimulation comprend la stimulation d'un ou plusieurs des sites d'un sous-ensemble séquentiellement en fonction d'une position au sein du groupement de zones de traitement.

9. Système (160) selon la revendication 1, dans lequel l'étape de stimulation comprend la stimulation d'un ou plusieurs des sites d'un sous-ensemble séquentiellement en fonction d'une valeur de mesure d'activité.

10. Système (160) selon la revendication 1, dans lequel l'étape de stimulation comprend la stimulation de deux ou de plus de deux des sites d'un sous-ensemble simultanément.

11. Système (160) selon la revendication 1, conçu en outre pour diviser le groupement de zones de traitement en un ou plusieurs segments, et dans lequel l'étape de détermination destinée à déterminer un sous-ensemble comprend la détermination d'au moins un site au sein du segment à inclure dans le sous-ensemble.

12. Système (160) selon la revendication 1, dans lequel l'appareil d'évaluation (162) sert en outre à rebalayer le premier sous-ensemble pour déterminer une deuxième mesure d'activité pour au moins un site parmi le premier sous-ensemble de sites.

13. Système (160) selon la revendication 1, dans lequel l'ordinateur (164) sert à recevoir les deuxièmes mesures d'activité, et à déterminer un deuxième sous-ensemble du premier sous-ensemble pour une simulation.

14. Système (160) selon la revendication 1, comprenant en outre un support non transitoire lisible par ordinateur, comprenant un logiciel exécutable par un processeur, et servant:
- à recevoir des valeurs de mesure d'activité en provenance d'un appareil d'évaluation (162), et à déterminer un ou plusieurs sous-ensembles d'une pluralité de sites d'un groupement de zones de traitement;
- le logiciel étant conçu pour effectuer les étapes suivantes:
- établissement d'un groupement de zones de traitement comportant une pluralité de sites;
- balayage d'une pluralité de sites du groupement de zones de traitement pour déterminer une valeur de mesure d'activité pour chaque site de la pluralité de sites du groupement de zones de traitement;
- détermination d'un premier sous-ensemble de la pluralité de sites du groupement de zones de traitement sur la base des valeurs de mesure d'activité;
- stimulation de chaque site au sein du premier sous-ensemble de la pluralité de sites;
- rebalayage de chaque site au sein du premier sous-ensemble de la pluralité de sites pour déterminer une deuxième valeur de mesure d'activité pour chaque site de la pluralité de sites stimulés au sein du premier sous-ensemble;
- détermination d'un deuxième sous-ensemble de la pluralité de sites du groupement de zones de traitement, le deuxième sous-ensemble de sites étant sélectionné dans le premier sous-ensemble de sites, et l'étape de détermination étant basée sur les deuxièmes valeurs de mesure d'activité obtenues après rebalayage du premier sous-ensemble de sites;
- stimulation de chaque site au sein du deuxième sous-ensemble de sites;
- rebalayage de chaque site au sein du deuxième sous-ensemble de la pluralité de sites pour déterminer une troisième valeur de mesure d'activité pour chaque site de la pluralité de sites stimulés au sein du deuxième sous-ensemble;
- détermination d'un troisième sous-ensemble de la pluralité de sites du groupement de zones de traitement, le troisième sous-ensemble de sites étant sélectionné dans le deuxième sous-ensemble de sites, et l'étape de détermination étant basée sur les troisièmes valeurs de mesure d'activité obtenues après rebalayage du deuxième sous-ensemble de sites.
